# EUROPEAN PATENT APPLICATION

(11) **EP 3 721 857 A1**
(43) Date of publication of application: **14.10.2020**
(21) Application number: 19168752.4
(22) Date of filing: 11.04.2019
(51) Int. Cl.: A61K 6/00

(54) **DENTAL COMPOSITION**

(71) Applicant: Dentsply DeTrey GmbH, 78467 Konstanz (DE)
(72) Inventor: Worm, Matthias, 78467 Konstanz (DE); Klee, Joachim E., 78315 Radolfzell (DE)
(74) Representative: Dietz, Mirko

(57) **Abstract**

Dental composition comprising
(i) one or more di- or polyepoxides;
(ii) a compound of the following formula (I)

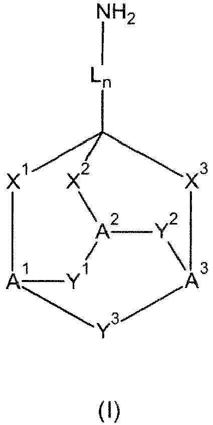

## Description

### Field of the Invention

The present invention relates to a dental composition comprising a specific primary amine compound. A dental composition in accordance with the invention is adapted to form epoxide-amine addition polymers. Furthermore, the present invention relates to a specific primary amine compound for use in the treatment or prevention of endodontic disease.

### Background of the Invention

Polymerizable dental compositions containing polymerizable compounds are known. Conventionally, polymerizable dental compositions are provided for a broad range of applications and must, therefore, meet diverse requirements. For example, a polymerizable dental composition may be a dental adhesive composition, a bonding agent, a pit and fissure sealant, a dental desensitizing composition, a pulp capping composition, a dental composite, a dental glass ionomer cement, a dental cement, a dental root canal sealer composition, a root canal filling composition, or a dental infiltrant.

Dental compositions are desired to approach natural tooth structure with regard to strength and appearance. Accordingly, a great effort is documented by the prior art, which is directed to the development of dental compositions having improved properties with regard to physical properties, biocompatibility, aesthetics and handling properties.

US 5,624,976 discloses a root canal sealing dental filling composition, wherein aniline, p-flouraniline, benzylamine, 1-aminoadamantane, α-phenylethylamine, dimethyl(amino)methyl) phosphine oxide and ethanolamine may be used as monoamine compounds.

Dental compositions selected from a root canal filling composition and a pulp capping composition are subject to additional requirements in that the cured product is required to have a high radiopacity and in that the composition may not require external irradiation for curing. Moreover, it is desirable that the composition adheres to the wall of the root canal in order to further improve the tight sealing of the dental root canal. Given that the shape of the root canal may change as a result to mastication and temperature changes, the cured composition must tolerate such changes without compromising a tight seal of the root canal. Accordingly, in order to provide such additional properties, a root canal filling composition or pulp capping composition contains radiopaque particulate fillers dispersed in a curable matrix. However, the dispersion of radiopaque particulate fillers gives rise to a stability problem of the dispersions due to the high density of the filler and the low viscosity of the curable matrix.

Moreover, in order to be able to cure a root canal filling composition or pulp capping composition in the absence of light, the composition is cured by a thermal curing mechanism which may involve step growth polymerizing epoxide precursor compounds.

Prior art dental filling materials for tooth root canals have relatively long setting time, high viscosity, and discolour.

The gold standard of dental root canal filling material, which presently offers the best overall properties, is AH Plus® from (Dentsply DeTrey, Konstanz/Germany). The good overall properties of AH Plus® especially concern physical and mechanical properties, curing properties like gelation time and handling properties like flow- and viscosity properties. The good overall properties highly depend on the used filler composition and the structure of the epoxide-amine addition polymer, which is formed during the curing process of the AH Plus® composition. The AH Plus® dental root canal filling composition consists of an amine paste and an epoxide paste. The AH Plus® epoxide paste comprises Bisphenol A diglycidylether (EP, CAS: 25068-38-6) as a main ingredient and the AH Plus® amine paste comprises the diamine N,N'-dibenzyl-5-oxanonandiamin-1,9 (DA, CAS: 113506-22-2) and the monoamine 1-aminoadamantane (MA, CAS: 768-94-5) as main ingredients. The molecular structure of these three main ingredients of AH Plus® is shown below.

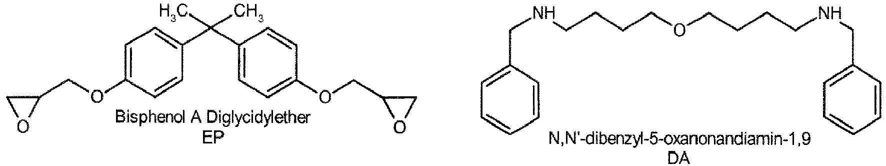

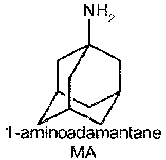

As it can be seen from the structures above, the three main ingredients EP, DA and MA of the AH Plus® composition have mirror-symmetrical molecular structures. In particular, the monoamine 1-aminoadamantane (MA) has a very high level of symmetry. Due to the fact that the addition polymerization method is a statistical polymerization method and that the monomers used in AH Plus® are mainly mirror-symmetrical monomers, a large number of mirror-symmetrical subunits in the polymer structure are formed during the polymerization step. These mirror-symmetrical subunits have a significant influence on the properties of the formed polymer. The smallest possible mirror-symmetrical subunits of the polymer leaving aside different diastereoisomers are shown below, but also larger mirror-symmetrical subunits in the polymer formed during the curing of AH Plus® may be possible but are not shown. However, in general 8 mirror-symmetrical subunits are possible.

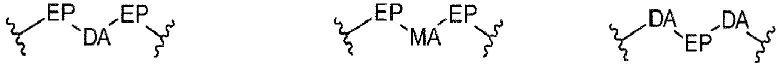

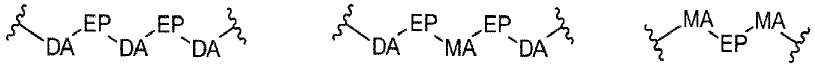

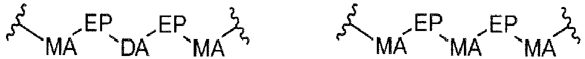

The monomers used in AH Plus® EP, DA and MA are relatively large monomers and the mirror-symmetrical subunits therefore make up relatively large blocks of the final polymer. For this reason, the mirror-symmetrical subunits have a large impact on the physical and mechanical properties of the final polymer and in particular the viscosity and the flow properties depend on the mirror-symmetrical subunits. Consequently, the mirror-symmetrical subunits contribute a lot to the overall properties of AH Plus.

### Summary of the Invention

It is the problem of the present invention to provide a dental composition having excellent properties with regard to physical and mechanical properties, biocompatibility, aesthetics and handling properties.

It is a further problem of the present invention to provide a dental composition having a high radiopacity, a high storage stability, a low shrinkage and flexibility, a relatively short setting time, and which may be cured in the absence of light.

Furthermore, it is a problem of the present invention to provide a dental composition which has adjustable working and setting times, suitable viscosity, and which shows no coloration problems.

It is a further problem of the present invention to provide a curable dental composition selected from a root canal filling composition and a pulp capping composition which is cost efficient, simple and available on a scale which is industrially relevant.

It is a further problem of the present invention to provide a dental composition which has comparable and/or improved overall properties compared to the AH Plus® composition.

It was surprisingly found that the problems of the present invention can be solved by a dental composition according to the present invention comprising
(i) one or more di- or polyepoxides;
(ii) a compound of the following formula (I) 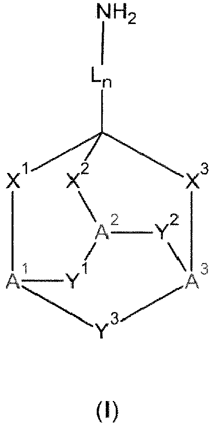 wherein
   L is a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
   at least one of A¹, A², and A³, represents CH, and the remaining groups are a group C-R¹;
   at least three of X¹, X², X³, Y¹, Y², and Y³, represent CH₂, and the remaining groups are a CHR²-group, a CR³R⁴-group, or a C=NR⁵-group; and
   n is 0 or 1;
      wherein
   R¹ is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
   R² is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
   R³ and R⁴ which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
   R⁵ is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C7-14 arylalkoxy group;
   provided that when n is 0, then at least one of R¹, R², R³/R⁴ and R⁵, is present.

Surprisingly, it was found that by using an unsymmetrical primary amine monomer according to formula (I) a dental composition can be provided which has comparable and/or improved overall properties compared to the AH Plus® composition. By using the unsymmetrical primary amine monomer according to formula (I) the number of possible mirror-symmetrical subunits decreases from 8 to 3 and therefore the statistically formed mirror-symmetrical subunits in the final polymer are decreased by 62.5%. Therefore, it was surprisingly found that the decrease of 62.5% of the mirror-symmetrical subunits in the final polymer of the cured dental composition, which have a big influence on the overall polymer properties, results in a dental composition having comparable and/or improved overall properties compared to the AH Plus® composition

Additionally, the present invention provides a dental composition which further comprises a radiopaque particulate filler.

Moreover, the present invention provides a dental composition, which is a root canal filling composition or a pulp capping composition.

Furthermore, the present invention provides a dental composition, which has a gel time of at most 20 hours.

Finally, the present invention provides a compound of the following formula (I) for use in the treatment or prevention of endodontic disease: 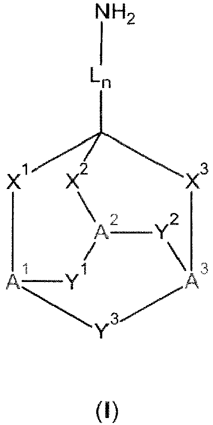 wherein
L is a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
at least one of A¹, A², and A³, represents CH, and the remaining groups are a group C-R¹; at least three of X¹, X², X³, Y¹, Y², and Y³, represent CH₂, and the remaining groups are a
CHR²-group, a CR³R⁴-group, or a C=NR⁵-group; and
n is 0 or 1;
wherein
- R¹: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
- R²: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
- R³ and R⁴: which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
- R⁵: is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group;
provided that when n is 0, then at least one of R¹, R², R³/R⁴ and R⁵, is present.

The present invention is based on the recognition that a dental composition comprising a compound of formula (I) provides a dental composition which has comparable and/or improved overall properties compared to the AH Plus® composition. Moreover, the present invention is based on the recognition that a dental composition comprising a compound of formula (I) provides a dental composition having excellent properties with regard to physical and mechanical properties, biocompatibility, aesthetics and handling properties, having a high radiopacity, a high storage stability, a low shrinkage and flexibility, a relatively short setting time, and may be cured in the absence of light. Furthermore, the dental composition according to the present invention provides adjustable working and setting times, suitable viscosity, and shows no coloration problems. Finally, the present invention provides a curable dental composition selected from a root canal filling composition and a pulp capping composition which is cost efficient, simple and available on a scale which is industrially relevant.

A primary monoamine monomer according to formula (I) according to the present invention is adapted to act as a monomer which offers excellent reactivity, so that a dental composition having excellent curing properties is provided. Furthermore, the use of the primary monomer according to formula (I) according to the present invention leads to a polymer having a lower number of mirror-symmetrical subunits compared to the final polymer of the AH Plus® composition, but has comparable and/or improved overall properties.

### Brief description of the Figures

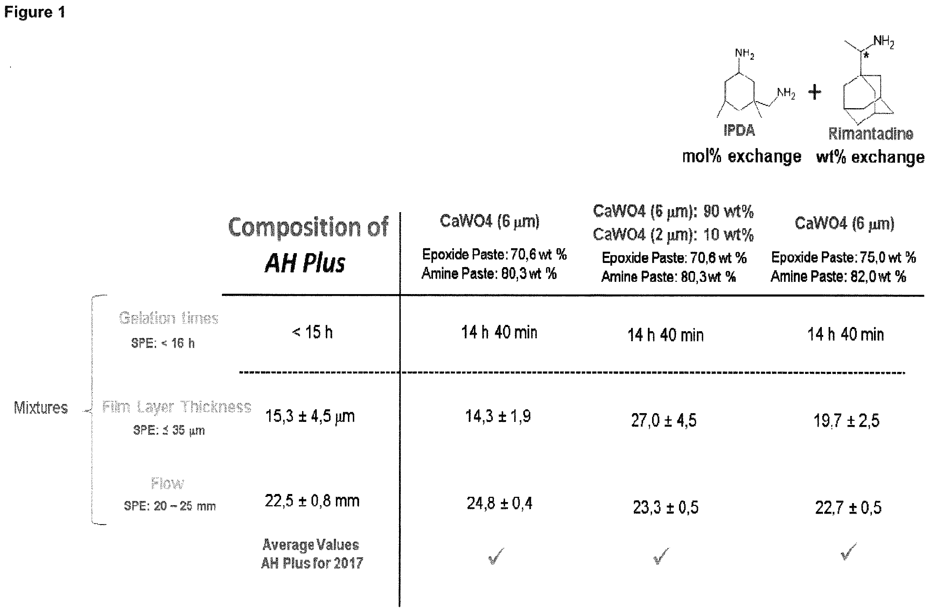

Fig. 1 shows a summary of the experimental results further disclosed in the Examples.

### Detailed description of preferred embodiments

The present invention relates to a dental composition comprising a specific primary amine compound. A dental composition in accordance with the invention is adapted to form epoxide-amine addition polymers. The dental composition includes di- or polyepoxides and a primary monoamine according to formula (I) and/or other monoamines and/or secondary diamines and/or a filler. The dental composition in accordance with the present invention is polymerizable and polymerizes to form a thermoplastic polymer.

Furthermore, the present invention relates to a specific primary amine compound for use in the treatment or prevention of endodontic disease.

The terms "polymerization" and "polymerizable" relate to the combining by covalent bonding of a large number of smaller molecules, such as monomers, to form larger molecules, that is, macromolecules or polymers. The monomers may be combined to form only linear macromolecules or they may be combined to form three-dimensional macromolecules, commonly referred to as crosslinked polymers. For example, difunctional monomers form linear polymers, whereas monomers having at least three functional groups form crosslinked polymers also known as networks. In case of a higher conversion rate of the polymerizable monomer, the amount of multifunctional monomers may be reduced or the leaching problem may be alleviated.

The term "curing" means the polymerization of functional polymerizable compounds such as monomers, oligomers or even polymers, into a polymer network, preferably a crosslinked polymer network.

The term "curable" refers to dental composition that will polymerize upon mixing.

The "working time" is the time between the beginning of the setting reaction when the polymer and modified particulate reactive filler are combined in the presence of water, and the time the setting reaction proceeds to the point when it is no longer practical to perform further physical work upon the system, e.g. spatulate it or reshape it, for its intended dental or medical application.

The "setting time" is the time measured from the beginning of the setting reaction in a restoration to the time sufficient hardening has occurred to allow subsequent clinical or surgical procedures to be performed on the surface of the restoration. In a setting reaction, due to the presence of polymerizable groups, a polymerization reaction takes place. In addition to the polymerization reaction, in a glass ionomer cement, the setting reaction additionally involves neutralization of acid groups, for example of polymerizable compounds, by a base in the form of a reactive particulate glass.

The term "storage stability" as used herein means that the dental composition keeps its characteristics, in particular its working time and setting time, even after a long storage time of for example about 2 years.

### The compound according to formula (I)

The dental composition of the present invention comprises a compound of the following formula (I): 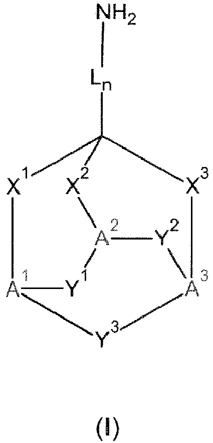 wherein
L is a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
at least one of A¹, A², and A³, represents CH, and the remaining groups are a group C-R¹;
at least three of X¹, X², X³, Y¹, Y², and Y³, represent CH₂, and the remaining groups are a group CHR², CR³R⁴ or C=NR⁵; and
n is 0 or 1;
wherein
- R¹: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
- R²: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
- R³ and R⁴: which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
- R⁵: is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group;
provided that when n is 0, then at least one of R¹, R², R³/R⁴ and R⁵, is present.

In a compound of formula (I), L represents a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group. Preferably, L represents a straight-chain hydrocarbon group having 1 to 8 carbon atoms, more preferably L represents a straight-chain hydrocarbon group having 2 to 6 carbon atoms, even more preferably L represents a straight-chain hydrocarbon group having 2 to 4 carbon atoms, and most preferable L represents a straight-chain hydrocarbon group having 2 carbon atoms.

L may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group. Preferably, L is substituted by one or more fluorine atoms, a hydroxyl group, or L is unsubstituted, more preferably L is substituted by a hydroxyl group or L is unsubstituted, and even more preferably L is unsubstituted.

In a particularly preferred embodiment, L represents the following group:

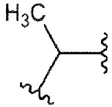

In a compound of formula (I), at least one of A¹, A², and A³, represents CH, and the remaining groups are a group C-R¹. Preferably, at least two or three of A¹, A², and A³, represent CH, and the remaining group is a group C-R¹, and more preferably at least three of A¹, A², and A³ represent CH.

In a compound of formula (I), at least three of X¹, X², X³, Y¹, Y², and Y³, represent CH₂, and the remaining groups are a group CHR², CR³R⁴ or C=NR⁵. Preferably, at least four of X¹, X², X³, Y¹, Y², and Y³, represent CH₂, and the remaining groups are a group CHR², CR³R⁴ or C=NR⁵, more preferably at least five of X¹, X², X³, Y¹, Y², and Y³, represent CH₂, and the remaining group is a group CHR², CR³R⁴ or C=NR⁵, most preferable at least six of X¹, X², X³, Y¹, Y², and Y³, represent CH₂.

Preferably, at least three, or at least four, or at least five of X¹, X², X³, Y¹, Y², and Y³, represent CH₂ and the remaining groups are a group of CHR², CR³R⁴, more preferably the remaining groups are a group of CHR².

In a preferred embodiment, the compound of formula (I) is a compound, wherein A¹, A², and A³, represent CH, and X¹, X², X³, Y¹, Y², and Y³ represent CH₂.

In a compound of formula (I), n is 0 or 1. Preferably, n is 1. However, in one embodiment, the compound of formula (I) is a compound, wherein n is 0. If n is 0, then at least one of R¹, R², R³/R⁴ and R⁵, is present.

In a compound of formula (I), R¹ is a fluorine atom, a hydroxyl group, an amino group, a C₁₋₆ alkyl group, a C1-6 alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group. Preferably, R¹ is a fluorine atom, a hydroxyl group, an amino group, a C₁₋₆ alkyl group, a C₁₋₆ alkoxy group, more preferably R¹ is a fluorine atom, a hydroxyl group, an amino group, a C₁₋₆ alkyl group, even more preferably R¹ is a fluorine atom, a hydroxyl group, a C₁₋₆ alkyl group, in particular R¹ is a hydroxyl group.

In a compound of formula (I), R² is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group. Preferably, R² is a fluorine atom, a hydroxyl group, an amino group, a C₁₋₆ alkyl group, a C₁₋₆ alkoxy group, more preferably R² is a fluorine atom, a hydroxyl group, an amino group, a C₁₋₆ alkyl group, even more preferably R² is a fluorine atom, a hydroxyl group, a C₁₋₆ alkyl group, in particular R² is a hydroxyl group.

In a compound of formula (I), R³ and R⁴ which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;

In a compound of formula (I), R⁵ represents a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group. Preferably, R⁵ represents a C₁₋₆ alkyl group, a C₆₋₁₀ aryl group, or C₇₋₁₄ arylalkyl group, more preferably R⁵ represents a C₁₋₆ alkyl group, or a C₆₋₁₀ aryl group, and even more preferably R⁵ represents a C₁₋₆ alkyl group.

In a particularly preferred embodiment, the compound of formula (I) is the following compound:

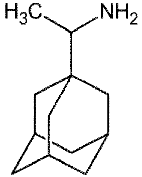

### The one or more di- or polyepoxides

The dental composition according to the present invention comprises one or more di- or polyepoxides. The one or more di- or polyepoxides may be any di- or polyepoxides suitable in dental applications and/or known in the art.

In a specific embodiment, the one or more di- or polyepoxides are compound of the following formula (II): 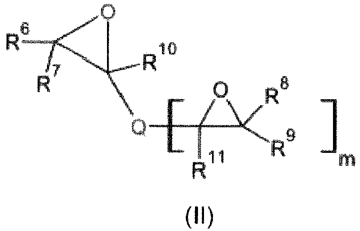 wherein
Q is an (m+1)-valent organic group,
R⁶, R⁷ and R¹⁰ which may be the same or different and which are independent from each other, represent a hydrogen atom, or a C₁₋₆ alkyl group,
the R⁸, R⁹ and R¹¹ which may be the same or different and which are independent from each other, represent a hydrogen atom, or a C₁₋₆ alkyl group,
m is an integer of from 1 to 3.

In a compound of formula (II), Q represents an (m+1)-valent organic group. Preferably, Q represents a 2-valent organic group, or a 3-valent organic group, and more preferably, Q represents a 2-valent organic group.

The (m+1)-valent organic group, may be a group having a total of 1 to 40 carbon atoms, preferably 2 to 20 carbon atoms. The organic group may include an aliphatic, alicyclic, or aromatic moiety or a combination of two or more of such moieties. The organic group may further include one or more functional groups such as amide groups, ester groups, urethane groups, urea groups, keto groups, ether groups, thioether groups, carbonate groups, or tertiary amino groups, which link two or more aliphatic, alicyclic, or aromatic moieties. Furthermore, the organic group may be substituted by one or more substituents selected from hydroxyl groups, halogen atoms, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group.

Preferably, the (m+1)-valent organic group may include alicyclic or aromatic moiety, and more preferably the (m+1)-valent organic group includes an aromatic moiety.

In a preferred embodiment, Q represents the following group:

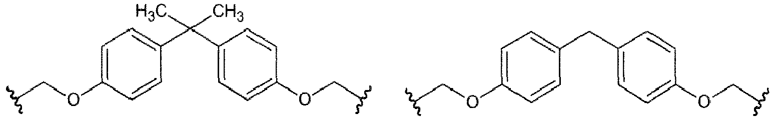

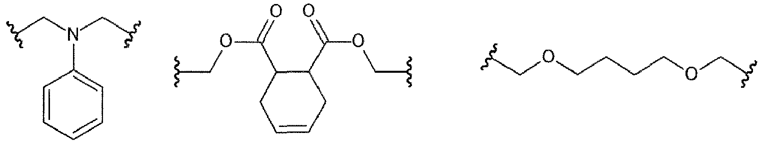

In a compound of formula (II), m is an integer of from 1 to 3. Preferably, m is an integer of from 1 to 2, and more preferably m is 1.

In a compound of formula (II), R⁶, R⁷ and R¹⁰ which may be the same or different and which are independent from each other, represent a hydrogen atom, or a C₁₋₆ alkyl group. Preferably, R⁶, R⁷ and R¹⁰ represent a hydrogen atom. In a particularly preferred embodiment, all three of R⁶, R⁷ and R¹⁰ represent a hydrogen atom.

In a compound of formula (II), the R⁸, R⁹ and R¹¹ which may be the same or different and which are independent from each other, represent a hydrogen atom, or a C₁₋₆ alkyl group. Furthermore, in a compound of formula (II), if more than one R⁸ is present, the more than one R⁸ may be different. In a compound of formula (II), if more than one R⁹ is present, the more than one R⁹ may be different. In a compound of formula (II), if more than one R¹¹ is present, the more than one R¹¹ may be different. Preferably, R⁸, R⁹ and R¹¹ represent a hydrogen atom. In a particularly preferred embodiment, all of the R⁸, R⁹ and R¹¹ moieties which are present are hydrogen atoms.

In particularly preferred embodiment, the compound according to formula (II) is Bisphenol A Diglycidylether (CAS: 25068-38-6), or Bis-[4-(-2,3-epoxypropoxy)phenyl]-methane (CAS: 9003-36-5).

### Further components

### Aliphatic polyamines

The dental composition according to the present invention, may further comprise an aliphatic polyamine. The aliphatic polyamine may be any aliphatic polyamine which is suitable in dental applications and/or known in the art.

In a specific embodiment, the aliphatic polyamine is selected among compounds of the following structures: 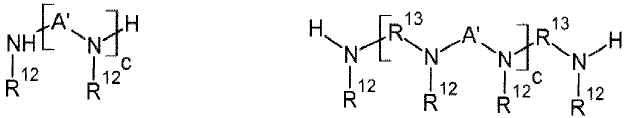 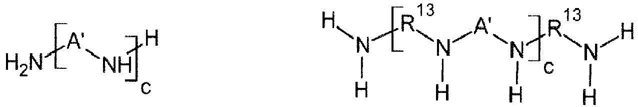 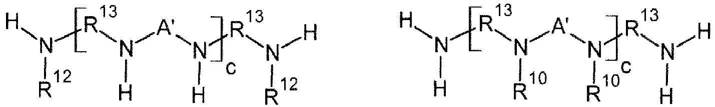 wherein
- R¹²: is hydrogen or a substituted or unsubstituted C₁₋₁₈ alkyl group, a substituted or unsubstituted C₃₋₁₈ cycloalkyl group, or a substituted or unsubstituted C₇₋₁₈ arylalkyl group,
- R¹³: is a difunctional substituted or unsubstituted C₁ to C₁₈ alkylene group, or a substituted or unsubstituted cycloalkylene group,
- A': is a moiety derived from a compound that is capable of an addition reaction with amines such as di- or polyepoxides, and
- c: is an integer.

In an aliphatic polyamine according to the structures above, preferably R¹² is hydrogen, a substituted or unsubstituted C₃₋₁₈ cycloalkyl group, or a C₇₋₁₈ arylalkyl group. More preferably, R¹² is a substituted or unsubstituted C₃₋₁₈ cycloalkyl group, or a substituted or unsubstituted C₇₋₁₈ arylalkyl group. Even more preferably, R¹² is a substituted or unsubstituted C₇₋₁₈ arylalkyl group. In a particularly preferred embodiment, R¹² is an unsubstituted C₇₋₁₈ arylalkyl group.

In an aliphatic polyamine according to the structures above, preferably R¹³ is difunctional substituted or unsubstituted C₁ to C₁₈ alkylene group, more preferably R¹³ is a difunctional unsubstituted C₁ to C₁₈ alkylene group.

In an aliphatic polyamine according to the structure above, A' is a moiety derived from a compound that is capable of an addition reaction with amines such as di- or polyepoxides. Preferably, A' is a moiety derived from an addition reaction of a di- or polyepoxide according to formula (II) and an amine. More preferably, A' is a moiety derived from an addition reaction of an amine with a compound of formula (II) which is a diepoxide.

In a preferred embodiment, A' is a moiety derived from an addition reaction of an amine and a compound of formula (II) which is a diepoxide, and wherein in the compound of formula (II) Q represents the following structures.

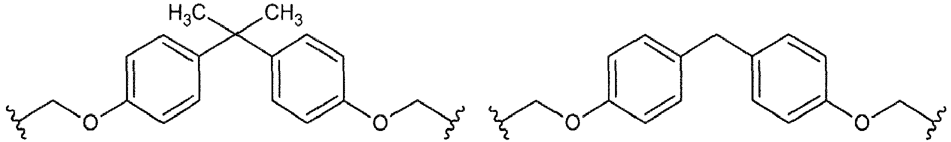

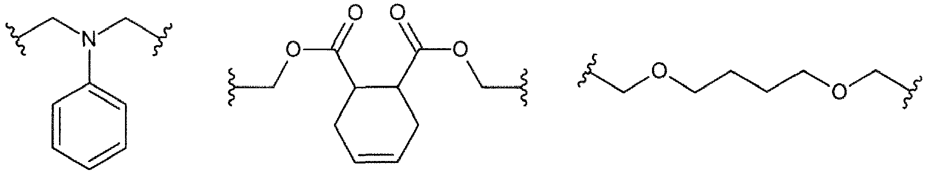

In an aliphatic polyamine according to the structure above, c is an integer. Preferably, c is an integer of from 1 to 10, more preferably c is an integer of from 2 to 8, even more preferably c is an integer of from 4 to 6.

### The Filler

The dental composition according to the present invention, may further comprise a filler. The filler in the dental composition according to the present invention may be any filler which is suitable for dental applications and/or known in the art.

In a specific embodiment, the filler according to the present invention is a radiopaque particulate filler. The radiopaque filler according to the present invention may be any radiopaque filler suitable for dental applications and/or known in the art.

The radiopaque particulate filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The radiopaque particulate filler may be a multimodal radiopaque particulate filler representing a mixture of two or more radiopaque particulate fractions having different average particle sizes. The radiopaque particulate filler may also be a mixture of particles of different chemical composition.

The radiopaque filler in the form of a particulate filler or a nanofiller may be selected from any of the zinc, ytterbium, yttrium, gadolinium, zirconium, strontium, tungsten, tantalum, thorium, niobium, barium, bismuth, molybdenum and lanthanum metals, alloys thereof, organometallic complexes thereof, oxides, sulfates, carbonates, halides, oxy-halides, subnitrates, tungstates and carbides thereof, iodine and inorganic iodides, either singly or in combination. In a preferred embodiment, the radiopaque filler is selected from any of bismuth trioxide, bismuth carbonate, bismuth oxy-chloride, bismuth subnitrate, zirconium oxide, barium sulfate, barium tungstate and calcium tungstate, either singly or in combination. In an even more preferred embodiment, the radiopaque filler is selected from barium tungstate and calcium tungstate, either singly or in combination. Preferably the radiopaque filler is calcium tungstate.

The dental composition according to the present invention preferably comprises 1 to 85 percent by weight, more preferably 40 to 85 percent by weight, even more preferably 40 to 70 percent by weight, of the radiopaque particulate filler, based on the weight of the entire composition.

The viscosity and thixotropicity of the uncured as well as the physical properties of the cured compositions may be controlled by varying the sizes and surface areas of the filler.

The filler may be surface treated with one or more silanating agents. Preferred silanating agents include those having at least one polymerizable double bond and at least one group that easily hydrolyses with water. Examples of such agents include 3-methacryloxypropyltrimethoxysilane, 3-methacryloxypropyldimethoxy-monochlorosilane, 3-methacryloxypropyld ichloromonomethoxysilane, methacryloxypropyltri-chlorosilane, 3-methacryloxypropyldichloromonomethyl-silane, 3-methacryloxypropylmonochlorodimethylsilane, or 2,3-epoxypropyltrimethoxysilane, aminopropyltrimethoxysilane, mercaptopropyltrimethoxysilane and mixtures thereof.

### Aliphatic mono- and diamines

The dental composition according to the present invention, may further comprise aliphatic mono- and/or diamines. The aliphatic mono- and/or diamines may be any aliphatic mono- and/or diamine which is suitable in dental applications and/or known in the art.

In a preferred embodiment, the dental composition according to the present invention comprises a monoamine selected from the group consisting of benzylamine, 1-aminoadamantane, α-phenylethylamine, dimethyl(aminomethyl) phosphine oxide and ethanolamine.

In a preferred embodiment, the dental composition according to the present invention comprises a diamine selected from the group consisting of N,N'-dibenzylethylenediamine, N,N'-dibenzyl-3,6-dioxaoctandiamine-1,8, N,N'-dibenzyl-5-oxanonandiamine-1,9 (CAS: 113506-22-2), N,N'-dibenzyl-(2,2,4)trimethylhexamethylendiamine, N,N'-dibenzyl-(2,4,4)trimethylhexamethylendiamine, N,N'-dibenzylcyclohexylenediamine, N,N'-dibenzyl-xylylenediamine.

In a particularly preferred embodiment, the dental composition according to the present invention comprises one or more primary aliphatic diamines. Preferably, the dental composition comprises one primary aliphatic diamine.

Preferably, the primary aliphatic diamines are compounds according to the following formula (III): 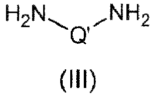 Wherein
- Q': represents a substituted or unsubstituted C₃₋₂₀ alkylene group or a substituted or unsubstituted C₃₋₂₀ cycloalkylene group, wherein the substituted C₃₋₂₀ alkylene group and the substituted C₃₋₂₀ cycloalkylene group may be substituted by one or more fluorine atoms, hydroxy groups, C₁₋₆ alkyl groups, C₃₋₁₂ cycloalkyl groups, a C₁₋₆ alkoxy groups, a C₁₋₆ alkylthio groups, a C₆₋₁₀ aryl groups, a C₆₋₁₀ aryloxy groups, a C7-14 arylalkyl groups, or a C₇₋₁₄ arylalkoxy groups.

In a compound of formula (III), preferably Q' represents a substituted or unsubstituted C₃₋₂₀ cycloalkylene group, more preferably Q' represents a substituted cycloalkylene group, and even more preferably Q' represents a substituted cycloalkylene group, wherein the substituents may be one or more C₁₋₆ alkyl groups, C₃₋₁₂ cycloalkyl groups.

In a particularly preferred embodiment, the dental composition according to the present invention comprises a primary aliphatic diamine, which is selected from the group consisting of octahydro-4,7-methano-1H-indenedimethylamine (CAS: 68889-71-4) and isophorone diamine (CAS: 2855-13-2).

### Further optional components

The dental composition according to the present invention may, besides of the above described components, comprise additional optional components. The dental composition according to the present invention may optionally contain any additive suitable for dental applications and/or known in the art.

For example, the dental composition according to the present invention may comprise water, or any solvent known in the art. The dental composition of the present invention may preferably comprise 5 to 20 percent by weight based on the total weight of the composition of water or any solvent known in the art.

Optionally, the dental composition may further comprise stabilizer(s), and/or pigments.

Preferably, the dental composition according to the present invention is a root canal filling composition or a pulp capping composition. More preferably, the dental composition according to the present invention is a root canal filling composition.

The dental composition according to the present invention has a gel time of at most 20 hours. Preferably, the dental composition according to the present invention has a gel time of at most 18 hours, more preferably the dental composition according to the present invention has a gel time of at most 15 hours.

Furthermore, the present invention provides the compound of formula (I) for use in the treatment or prevention of endodontic disease. 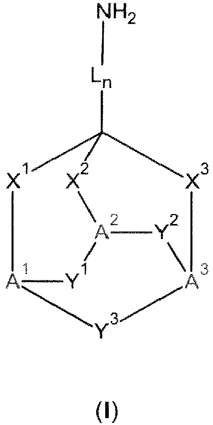 wherein
L is a straight-chain or branched hydrocarbon group having 1 to 8 carbon atoms, which may be substituted by one or more fluorine atoms, a hydroxyl group, C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
at least one of A¹, A², and A³, represents CH, and the remaining groups are a group C-R¹;
at least three of X¹, X², X³, Y¹, Y², and Y³, represent CH₂, and the remaining groups are a group CHR² group, CR³R⁴ or C=NR⁵; and
n is 0 or 1;
wherein
- R¹: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
- R²: is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
- R³ and R⁴: which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
- R⁵: is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group;
provided that when n is 0, then at least one of R¹, R², R³/R⁴ and R⁵, is present.

The invention will now be further illustrated by the following Examples.

### Examples

### Materials

Rimantadine (1-(1-adamantyl)ethylamine) hydrochloride was purchased from *abcr* and converted into the free base by stirring the hydrochloride in mixture of diethyl ether and sodium hydroxide solution (2N) followed by extraction with diethyl ether. After removal of the solvent under reduced pressure, the free rimantadine base was obtained in quantitative yield. Bisphenol A diglycidyl ether (Araldite GY 250, CAS 25068-38-6) and bisphenol F diglycidyl ether (Araldite GY 285, CAS 9003-36-5). Isophorone diamine (IPDA) was purchased from *Sigma Aldrich.* N,N'-Dibenzyl-5-oxanonandiamin-1,9 (OPC-91) was obtained from *A.M.B. LIFE & SIENCE ApS.* CaWO₄ (1 µm and 6 µm Grade B) particles were purchased from *Starck H.C. GmbH.* Aerosil₂₀₀ was provided by *CSC Jäkle Chemie.* SICOVIT® (Yellow 10 E 172) was purchased from *Simon und Werner GmbH.* All other chemicals were purchased from common chemical suppliers.

### Gelation time [Method]

Amine pastes and epoxide pastes were mixed (m_{Amine}/m_{Epoxide}, mixing ratio are given for each application example) on a mixing plate using a spatula. Mixing was applied for 30 s until a homogenous paste-paste-mixture was accomplished. The respective mass of the individual pastes was determined by means of a balance with an accuracy of ±0.0001 g. Approx. 6 g of the paste-paste mixture was transferred into a glass vial (10 mL) with a round-cut plastic lid and glass bar. The glass bar needs to be in contact with the paste mixture. The vial was placed in a climate chamber at 37°C (to) with a temperature control [ΔT = ± 1°C] and a relative humidity of 30 - 50%. Gelation was achieved when rotation of the glass bar was no longer possible (t_{gelation}).

### Paste Formulations

### AH Plus® Reference

AH Plus amine paste (Batch number: 1901000196) and AH Plus epoxide paste (Batch number: 1812100076) were mixed in a ratio m_{Amine}/m_{Epoxide} = 0.86207 The mixed pastes exhibit a gelation time of less than 16 h (method described above), flow of 23 ± 0 mm (ISO 6876:2012) and a film thickness of 20 ± 8 µm (ISO 6876:2012).

### Paste A1.

### Preparation of Epoxide Paste A1 (SAR3-08-01)

Bisphenol A diglycidyl ether (7.7205 g), bisphenol F diglycidyl ether (0.9766 g), CaWO₄ Grade B (16.9399 g), CaWO₄ (1 µm) (4.2365 g) and SICOVIT® (Yellow 10 E172) (0.0438 g) were added and speed mixing was applied (1 min, 2150 rpm). Aerosil®200 (0.0850 g) was added and followed by a speed mixer run (1 min, 2150 rpm). The paste was manually mixed with a spatula to disperse filler residue from the container wall followed by another speed mixer run (1 min, 2150 rpm) to afford a homogenous, light yellow paste. Density (T = 23°C): 2,7277 g/mL (measured by means of a helium pycnometer).

### Preparation of Amine Paste A1 (SAR2-06-01)

Rimantadine (1-(1-adamantyl)ethylamine) (1.4835 g), N,N'-dibenzyl-5-oxanonandiamin-1,9 (OPC-91) (3.3351 g) and isophorone diamine (IPDA) (0,1983 g) were transferred into a speed mixer container and mixed for 5 min with 2150 rpm. CaWO₄ (1 µm) (4.8203 g) and CaWO₄ Grade B (19.2703 g) were added and speed mixing was applied (5 min, 2150 rpm, 1000 mBar). Aerosil®₂₀₀ (0.5847 g) and Baysilone M500 (0.3189 g) were added and the past was manually mixed with a spatula to disperse filler residue from the container wall followed by another speed mixer run (1 min, 2150 rpm) to afford a homogenous, white paste. Density (T = 23°C): 3.0609 g/mL (measured by means of a helium pycnometer).

### Mixtures of Paste A1 (SAR3-06-01+SAR3-08-01)

Amine Paste A1 and epoxide Paste A1 were mixed in a ratio m_{Amine}/m_{Epoxide} = 0,89112. The mixed pastes exhibit a gelation time of less than 16 h (SAR3-25-01), flow of 23.3 ± 0.5 mm, film thickness of 27 ± 5 µm (all according to ISO 6876:2012).

### Paste A2.

### Preparation of Epoxide Paste A2 (SAR3-07-01)

Bisphenol A diglycidyl ether (6.5473 g), bisphenol F diglycidyl ether (0.8571 g), CaWO₄ Grade B (22.5025 g) and SICOVIT® (Yellow 10 E172) (0.0423 g) were added and speed mixing was applied (1 min, 2150 rpm). Aerosil®₂₀₀ (0.0844 g) was added and followed by a speed mixer run (1 min, 2150 rpm). The paste was manually mixed with a spatula to disperse filler residue from the container wall followed by another speed mixer run (1 min, 2150 rpm) to afford a homogenous, light yellow paste. Density (T = 23°C): 2.9568 g/mL (measured by means of a helium pycnometer).

### Preparation of Amine Paste A2 (SAR2-05-01)

Rimantadine (1-(1-adamantyl)ethylamine) (1.3287 g), N,N'-dibenzyl-5-oxanonandiamin-1,9 (OPC-91) (2.9887 g) and isophorone diamine (IPDA) (0,1936 g) were transferred into a speed mixer container and mixed for 5 min with 2150 rpm. CaWO₄ Grade B (24.5983 g) were added and speed mixing was applied (5 min, 2150 rpm, 1000 mBar). Aerosil®200 (0.5849 g) and Baysilone M500 (0.3165 g) were added and the past was manually mixed with a spatula to disperse filler residue from the container wall followed by another speed mixer run (1 min, 2150 rpm) to afford a homogenous, white paste. Density (T = 23°C): 3.2928 g/mL (measured by means of a helium pycnometer).

### Mixtures of Paste A2 (SAR3-05-01+SAR3-07-01)

Amine Paste A2 and epoxide Paste A2 were mixed in a ratio m_{Amine}/m_{Epoxide} = 0,89797. The mixed pastes exhibit a gelation time of less than 16 h (SAR3-24-02), flow of 22.7 ± 0.5 mm, film thickness of 20 ± 2 µm (all according to ISO 6876:2012).

## Claims

1. Dental composition comprising
(i) one or more di- or polyepoxides;
(ii) a compound of the following formula (I) 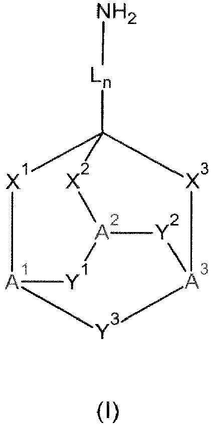 wherein
L is a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
at least one of A¹, A², and A³, represents CH, and the remaining groups are a group C-R¹;
at least three of X¹, X², X³, Y¹, Y², and Y³, represent CH₂, and the remaining groups are a CHR²-group, a CR³R⁴-group, or a C=NR⁵-group; and
n is 0 or 1;
wherein
R¹ is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
R² is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
R³ and R⁴ which may be the same or different, independent from each other represent a fluorine atom, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
R⁵ is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group;
provided that when n is 0, then at least one of R¹, R², R³/R⁴ and R⁵, is present.

2. The dental composition according to claim 1, wherein at least two of A¹, A², and A³ represent CH.

3. The dental composition according to claim 1 or 2, wherein at least five of X¹, X², X³, Y¹, Y², and Y³ represent CH₂.

4. The dental composition according to any one of the preceding claims, wherein n is 1.

5. The dental composition according to any one of the preceding claims, wherein L is the following group:

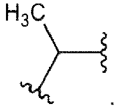

6. The dental composition according to any one of the preceding claims, wherein A¹, A², and A³, represent CH, and X¹, X², X³, Y¹, Y², and Y³ represent CH₂.

7. The dental composition according to any one of claims 1 to 3, wherein n is 0.

8. The dental composition according to claim 7, wherein R¹ is a flourine atom, a hydroxyl group, an amino group, a C₁₋₆ alkyl group, or a C₁₋₆ alkoxy group.

9. The dental composition according to claim 7 or 8, wherein R² is a flourine atom, a hydroxyl group, an amino group, a C₁₋₆ alkyl group, or a C₁₋₆ alkoxy group.

10. The dental composition according to any one of the preceding claims, wherein the one or more di- or polyepoxides are compounds of the following formula (II): 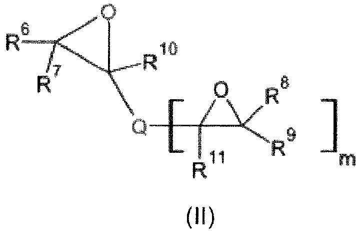 wherein
Q is a (m+1)-valent organic group,
R⁶, R⁷ and R¹⁰ which may be the same or different and which are independent from each other, represent a hydrogen atom, or a C₁₋₆ alkyl group,
the R⁸, R⁹ and R¹¹ which may be the same or different and which are independent from each other, represent a hydrogen atom, or a C₁₋₆ alkyl group,
m is an integer of from 1 to 3.

11. The dental composition according to any one of the preceding claims, which further comprises an aliphatic polyamine selected among compounds of the following structures: 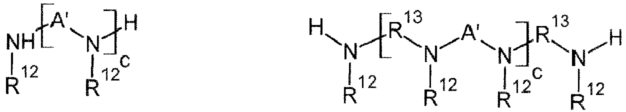 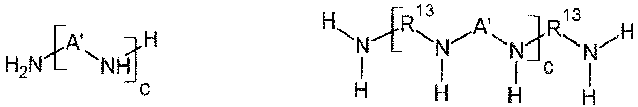 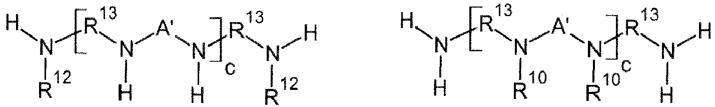 wherein
R¹² is hydrogen or a substituted or unsubstituted C₁₋₁₈ alkyl group, a substituted or unsubstituted C₃₋₁₈ cycloalkyl group, or a substituted or unsubstituted C₇₋₁₈ aryalkyl group,
R¹³ is a difunctional substituted or unsubstituted C₁ to C₁₈ alkylene group, or a substituted or unsubstituted cycloalkylene group,
A' is a moiety derived from a compound that is capable of an addition reaction with amines such as di- or polyepoxides, and
c is an integer.

12. The dental composition according to any one of the preceding claims, which further comprises a radio-opaque particulate filler.

13. The dental composition according to any one of the preceding claims, which is a root canal filling composition or a pulp capping composition.

14. The dental composition according to any one of the preceding claims, which has a gel time of at most 20 hours.

15. A compound of the following formula (I) for use in the treatment or prevention of endodontic disease: 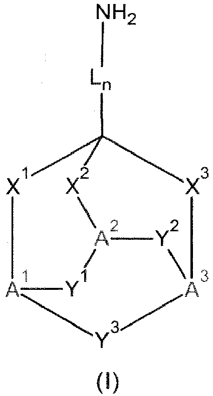 wherein
L is a straight-chain or branched hydrocarbon group having 1 to 8 carbon atoms, which may be substituted by one or more fluorine atoms, a hydroxyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
at least one of A¹, A², and A³, represents CH, and the remaining groups are a group C-R¹;
at least three of X¹, X², X³, Y¹, Y², and Y³, represent CH₂, and the remaining groups are a CHR²-group, a CR³R⁴-group, or a C=NR⁵-group; and
n is 0 or 1;
wherein
R¹ is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group; and
R² is a fluorine atom, a hydroxyl group, an amino group, a C₁₋₆ alkyl group, a C₁₋₆ alkoxy group, a C₁₋₆ alkylthio group, a C₆₋₁₀ aryl group, a C₆₋₁₀ aryloxy group, a C₇₋₁₄ arylalkyl group, or a C₇₋₁₄ arylalkoxy group;
R³ and R⁴ which may be the same or different, independent from each other represent a fluorine atom, a C₁₋₆ alkyl group, a C₁₋₆ alkoxy group, a C₁₆ alkylthio group, a C₆₋₁₀ aryl group, a C₆₋₁₀ aryloxy group, a C₇₋₁₄ arylalkyl group, or a C₇₋₁₄ arylalkoxy group, or R³ and R⁴ may be linked together and forming together with the carbon atom to which they are bonded a 3 to 6-membered saturated hydrocarbon ring;
R⁵ is a hydroxyl group, an amino group, a C₁₋₆ alkyl group, a C₁₋₆ alkoxy group, a C₆₋₁₀ aryl group, a C₆₋₁₀ aryloxy group, a C₇₋₁₄ arylalkyl group, or a C₇₋₁₄ arylalkoxy group;
provided that when n is 0, then at least one of R¹, R², R³/R⁴ and R⁵, is present.
